# FASCICULE DE BREVET EUROPEEN

(11) **EP 3 049 164 B1**
(45) Date de publication et mention de la délivrance du brevet: **09.08.2017**
(21) Numéro de dépôt: 14790164.9
(22) Date de dépôt: 31.07.2014
(51) Int. Cl.: A63B 24/00, A63B 39/00, A63B 43/00, A63B 37/02, A63B 37/10, A63B 37/00, F03G 7/08, A43B 7/04, B62M 3/08, A43B 3/00, A61N 1/378

(54) **DISPOSITIF A COQUE RIGIDE DESTINE A SUBIR DES CHOCS ET COMPRENANT DES MOYENS INTERNES DE RECUPERATION D'ENERGIE**
VORRICHTUNG MIT EINER STARREN SCHALE ZUM ABWEHREN VON STÖSSEN UND MIT EINEM INNEREN ENERGIERÜCKGEWINNUNGSMITTEL
DEVICE WITH RIGID SHELL DESIGNED TO UNDERGO IMPACTS AND COMPRISING INTERNAL ENERGY RECOVERY MEANS

(30) Priorité: 25.09.2013 FR 1359233
(43) Date de publication de la demande: 03.08.2016
(73) Titulaire: Commissariat à l'Énergie Atomique et aux Énergies Alternatives, 75015 Paris (FR)
(72) Inventeur: SAVELLI, Guillaume, F-38000 Grenoble (FR); CORONEL, Philippe, F-38530 Barraux (FR)
(74) Mandataire: Cabinet Laurent & Charras
(86) Numéro de dépôt international: PCT/FR2014/051995
(87) Numéro de publication internationale: WO 2015/044545

(56) Documents cités:
- EP-A1- 2 364 752
- US-A- 2 020 484
- US-A1- 2003 228 934
- US-A1- 2009 229 142
- US-B1- 7 614 959

## Description

### DOMAINE DE L'INVENTION

L'invention a trait au domaine de la fonctionnalisation d'objets rigides destinés à subir des chocs, notamment des balles, des ballons ou des palets, en particulier dans le domaine du sport et/ou de la rééducation physique.

### ETAT DE LA TECHNIQUE

Dans les sports de balle, de ballon et de palets à coque rigide et dans la rééducation physique à base de tels objets, il est utile de disposer de statistiques qui permettent aux joueurs d'analyser leur jeu et au personnel médical d'évaluer la qualité des exercices pratiqués par les patients. Usuellement, ces statistiques sont récoltées manuellement en comptant par exemple le nombre de frappes, rebonds ou autres qu'un joueur ou un patient exerce sur une balle pendant une durée prédéterminée.

En outre, certains sports consomment beaucoup de balles, ces dernières ayant une durée de vie limitée, balles qu'il est nécessaire de recycler, ce qui génère un coût non négligeable.

Il existe ainsi un intérêt à intégrer dans ces objets, et de manière plus générale dans tout objet rigide destiné à subir des chocs, des fonctions électroniques permettant par exemple de réaliser automatiquement des statistiques et/ou de convertir et stocker l'énergie mécanique prodiguée à ces objets lors de leur utilisation en énergie électrique.

On connait du document US 2011/136603 une balle de sport comportant une coque déformable délimitant un volume interne sous pression, comme par exemple une balle de tennis, et comportant un circuit de récupération d'énergie à base de matériau piézoélectrique, qui convertit une partie de l'énergie mécanique reçue par la coque sous l'effet de sa déformation par un impact, en énergie électrique, et qui stocke l'énergie électrique ainsi produite dans une batterie interne à la balle. L'énergie ainsi récupérée et stockée est utilisée par un circuit interne à la balle, comme par exemple un accéléromètre, un capteur de pression ou un système GPS.

Ce document est dédié aux balles et ballons à coque déformable et le matériau piézoélectrique est agencé sur ou dans la coque déformable. La coque souple étant déformable, le matériau piézoélectrique subit donc également des déformations, ce qui permet de produire une grande quantité de charges électriques. Cependant, à supposer que l'enseignement de ce document soit appliqué à un objet à coque rigide, par exemple un palet, le choc ne déformant pas l'objet, la production de charges électriques est alors induite par les ondes acoustiques qui se propagent depuis la coque rigide au matériau piézoélectrique. En outre, le document US 2011/136603 est muet sur les moyens d'intégrer ces éléments électriques et sur ce qu'il advient des circuits embarqués dans les balles et les ballons une fois ceux-ci hors d'usage.

En outre, la production d'énergie et l'intégration d'une fonction de récupération d'énergie et/ou d'une fonction électronique, comme par exemple une fonction de comptage, se pose dans les mêmes termes pour tout objet rigide destiné à subir des chocs ou des accélérations lors de son utilisation.

Le document EP2364752A1, qui est considéré comme l'état de la technique le plus proche de l'objet de la revendication 1, divulgue un dispositif comportant une coque rigide externe (342) délimitant un volume interne, tel qu'il comprend : une coque interne (346) délimitant un volume interne, ladite coque interne étant logée dans le volume interne de la coque externe, et comprenant au moins une couche de matériau piézoélectrique (344) apte à produire de l'énergie électrique sous l'effet d'un choc que celle-ci subit contre la coque externe; un circuit électrique (348) logé dans le volume interne de la coque interne, connecté électriquement (voir "connecting lead 350 ") à la couche de matériau piézoélectrique, et comportant un élément de stockage de l'énergie électrique (404) produite par la couche de matériau piézoélectrique de la coque interne; et un élément de maintien (352) du circuit électrique dans la coque interne.

### EXPOSE DE L'INVENTION

Le but de la présente invention est de proposer un dispositif rigide qui comprend des moyens de production d'énergie électrique efficaces.

A cet effet, l'invention a pour objet un dispositif comportant une coque rigide externe délimitant un volume interne creux, qui comprend :
▪ une coque interne rigide délimitant un volume interne creux, ladite coque interne étant logée dans le volume interne de la coque externe et libre de se déplacer librement dans celui-ci et comprenant au moins une couche de matériau piézoélectrique apte à produire de l'énergie électrique sous l'effet d'un choc que celle-ci subit contre la coque externe;
▪ un circuit électrique logé dans le volume interne de la coque interne, connecté électriquement à la couche de matériau piézoélectrique, et comportant un élément de stockage d'énergie électrique produite par la couche de matériau piézoélectrique de la coque interne; et
▪ des éléments de maintien du circuit électrique dans la coque interne.

Par « rigide », on entend ici une coque qui ne se déforme sensiblement pas sous l'effet d'impacts subis par celle-ci au cours d'une utilisation standard de la coque. Par « déformable », on entend un élément qui se déforme sous l'effet d'impacts que celui-ci subit au cours d'une utilisation standard.

Par « libre de se déplacer », on entend ici que l'objet interne n'est pas connecté, relié ou fixé, à la coque externe, ou bien connecté à cette dernière à l'aide d'un système de maintien appliquant une faible force de rappel s'opposant de manière minime aux déplacements de l'objet interne.

En d'autres termes, une cavité est prévue dans le dispositif rigide et la production d'énergie électrique est réalisée par un objet comportant une coque piézoélectrique et libre de se déplacer dans cette cavité. Sous l'effet d'un choc ou d'une accélération, l'objet interne frappe donc la paroi de la cavité, ce qui génère des charges électriques par effet piézoélectrique. En outre, l'objet peut subir des chocs plusieurs fois, ce qui augmente la quantité totale de charges électriques produites.

Selon un mode de réalisation, la paroi interne de la coque externe est munie de picots rigides en saillie régulièrement disposés sur ladite paroi. De cette manière, les picots excitent directement la coque piézoélectrique. Notamment, lorsque la coque piézoélectrique comporte une membrane piézoélectrique externe, les picots permettent une production accrue de charges électriques.

Selon un mode de réalisation, la coque interne est rigide. Plus particulièrement, la coque interne rigide est constituée d'un matériau piézoélectrique rigide, un tel matériau présentant des performances piézoélectriques supérieures à celle d'un matériau piézoélectrique souple.

En variante, une membrane piézoélectrique souple est plaquée contre la paroi externe de la coque interne rigide. Une telle membrane peut ainsi épouser la paroi externe de la coque interne, ce qui permet d'être libre de la géométrie de la coque interne. En outre, en étant placée sur la face externe de la coque, la membrane piézoélectrique subit directement les chocs, produisant de ce fait plus de charges électriques. Avantageusement, la coque interne rigide a une géométrie choisie de manière à être sensible dans toutes les directions. Notamment, la coque interne rigide est sphérique. En variante, le volume interne de la coque externe et la paroi externe de la coque interne présentent la même géométrie que celle de la paroi externe de la coque externe. De cette manière, les charges électriques produites représentent fidèlement les chocs subits par la coque externe. Par exemple, un palet de hockey sur glace comporte un volume interne de même et une coque interne ayant la même forme que le palet lui-même.

Avantageusement, la coque interne rigide forme un boitier pour le circuit électrique, et les éléments de maintien sont des éléments de fixation solidarisant le circuit électrique au boitier, ce qui permet d'obtenir un objet interne compact et simple de réalisation.

En variante, le circuit électrique comporte une structure rigide, et les éléments de maintien comportent des éléments élastiques longilignes aptes à maintenir le circuit électrique dans une position prédéterminée dans le volume interne de la coque interne, chaque élément longiligne de maintien étant disposé entre la structure rigide du circuit électrique et une paroi interne de la coque interne. De cette manière, le circuit est maintenu au centre de la coque interne et les éléments de maintien élastiques absorbent les chocs subis par la coque interne, protégeant de ce fait les circuits électroniques. Notamment, les éléments de maintien sont constitués de ressorts. Les ressorts présentent l'avantage de nécessiter un volume limité de matière pour mettre en oeuvre de manière efficace une force de rappel, et perturbent donc au minimum le fonctionnement, par exemple aérodynamique, du dispositif.

De manière avantageuse, la coque interne comporte une couche de matériau absorbant les chocs entre une couche de matériau piézoélectrique et le circuit électrique, par exemple une couche de mousse, de gel, de polyester, de PCM (acronyme de « *Phase change material* »), ou de papier, de manière à protéger le circuit électrique. Par exemple, la coque interne comprend deux parois rigides concentriques définissant un espace remplie d'un matériau absorbant les chocs.

Selon un mode de réalisation, au moins deux des éléments de maintien sont conducteurs de l'électricité et forment deux connexions électriques entre la couche piézoélectrique de la coque interne et le circuit électrique. Il n'est ainsi pas nécessaire de prévoir d'autres types de connexion électrique, comme par exemple des fils soudés. En outre, ces connexions résistent aux chocs.

Selon un mode de réalisation, le circuit électrique est constitué d'étages électriques parallélépipédiques agencés en parallèle dans un cadre rigide. Ce type de configuration permet d'obtenir un circuit compact, et donc perturbant de manière minime le fonctionnement du dispositif.

Selon un mode de réalisation, le dispositif comporte une membrane piézoélectrique plaquée contre la paroi interne de la coque externe et un circuit électrique fixé à la paroi interne de la coque externe, connecté électriquement à la membrane piézoélectrique de la coque externe, et comportant un élément de stockage d'énergie électrique produite par la membrane piézoélectrique de la coque externe. De cette manière, l'ensemble des éléments subissant des chocs produisent des charges électriques sous l'effet d'un choc ou d'une accélération, augmentant de ce fait la quantité de charges produites.

Selon un mode de réalisation, le circuit électrique comporte un circuit de production de données à partir de l'énergie électrique produite par la couche piézoélectrique de la coque interne, et un circuit de transmission sans fil desdites données à l'extérieur de la coque externe, lesdits circuits de production et de transmission étant alimentés par l'élément de stockage d'énergie électrique.

Selon un mode de réalisation, l'élément de stockage d'énergie électrique comporte une micro-batterie formée sur un substrat souple ou rigide et/ou une capacité et/ou une supercapacité. Ce type de stockage d'énergie électrique est très léger, usuellement d'un poids et d'une surface faibles pour une grande capacité de stockage.

De manière avantageuse, la coque interne est connectée à la coque externe au moyen d'un système de rappel de la coque interne dans une position prédéterminée du volume interne de la coque externe, ce qui permet de produire une charge électrique minimale quel que soit la direction d'un impact, d'une accélération ou d'une secousse subis par le dispositif.

Notamment, le dispositif est une balle, un ballon, un palet, une pédale de bicyclette, une semelle de chaussure, une rame, une voile, une crosse, une batte ou une raquette.

### BREVE DESCRIPTION DES FIGURES

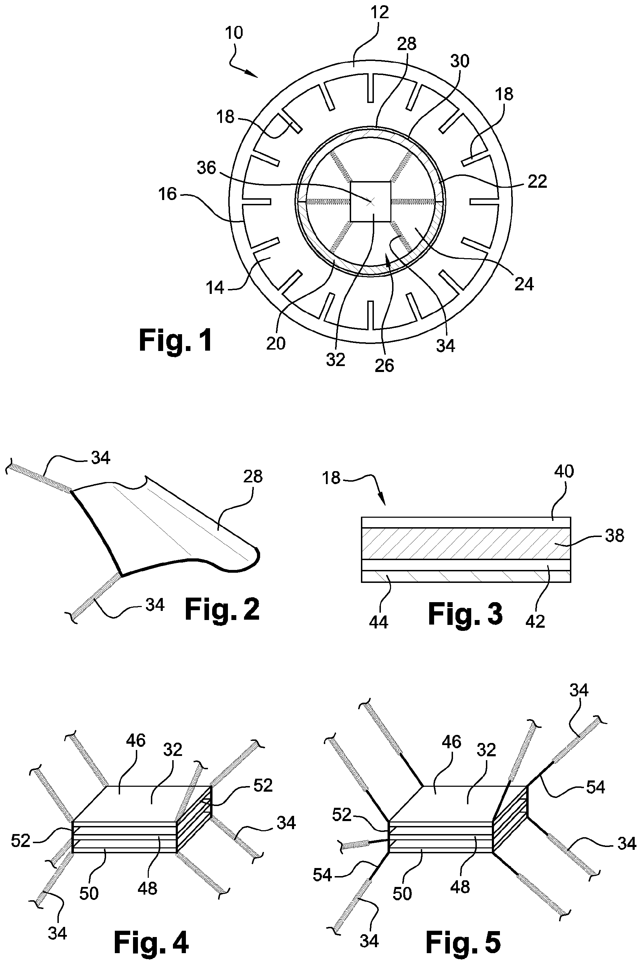

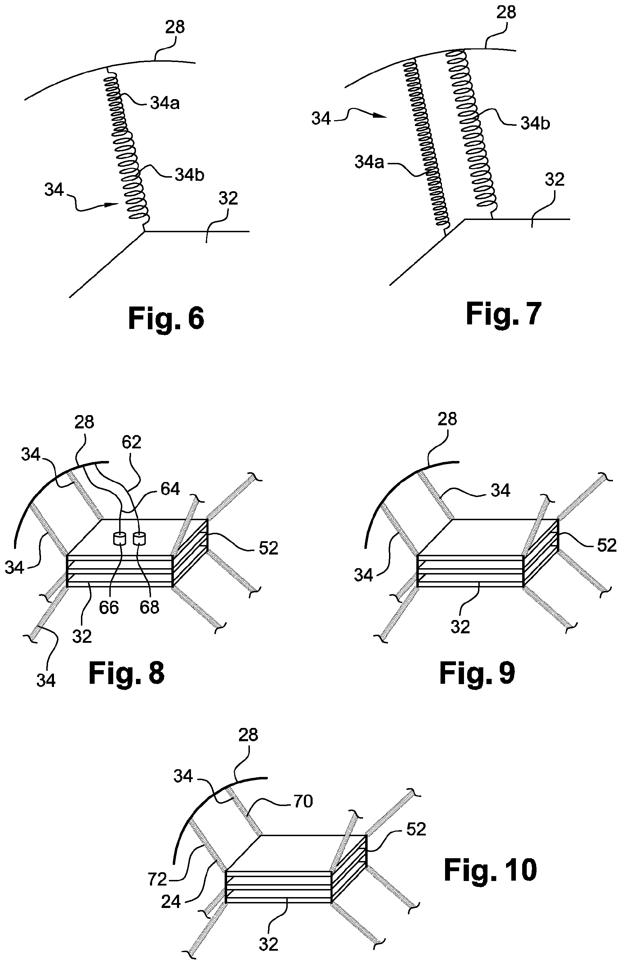

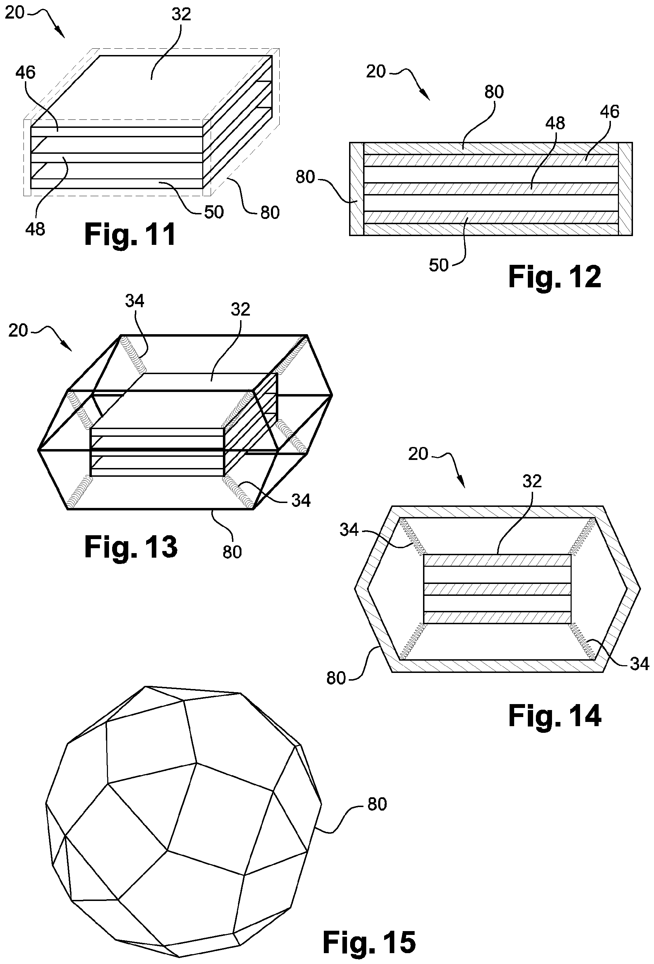

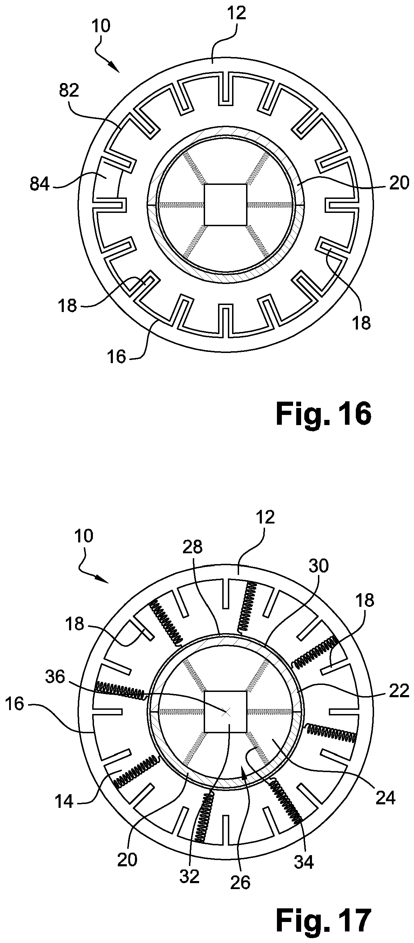

L'invention sera mieux comprise à la lecture de la description qui va suivre, donnée uniquement à titre d'exemple, et réalisée en relation avec les dessins annexés dans lesquels :
▪ la figure 1 est une vue en coupe schématique d'une balle conforme à un premier mode de réalisation de l'invention dans lequel un matériau piézoélectrique souple est utilisé;
▪ la figure 2 est une vue schématique en perspective d'une portion de la membrane piézoélectrique flexible et des éléments de maintien de la figure 1 ;
▪ la figure 3 est une vue schématique en coupe de la membrane piézoélectrique de la figure 1 ;
▪ la figure 4 est une vue schématique en perspective du circuit et des éléments de maintien électriques de la figure 1 ;
▪ la figure 5 est une vue schématique en perspective du circuit et des éléments de maintien électrique selon une variante de l'invention ;
▪ les figures 6 et 7 sont des vues schématiques des éléments de maintien selon deux variantes de l'invention ;
▪ les figures 8, 9 et 10 sont des vues schématiques de connexions électriques entre la membrane piézoélectrique et le circuit électrique de la figure 1 selon plusieurs variantes de l'invention ;
▪ les figures 11 et 12 sont respectivement des vues en perspective et en coupe d'un second mode de réalisation de l'invention dans lequel un matériau piézoélectrique rigide est utilisé ;
▪ les figures 13 et 14 sont respectivement des vues en perspective et en coupe d'une variante du second mode de réalisation ;
▪ la figure 15 est une vue en perspective d'une variante du second mode de réalisation ;
▪ la figure 16 est une vue en coupe d'un troisième mode de réalisation de l'invention dans lequel la paroi interne de la coque externe est piézoélectrique ; et
▪ la figure 17 est une vue en coupe d'une variante de l'invention comportant des ressorts de rappel de l'objet interne dans une position prédéterminée.

### DESCRIPTION DETAILLEE DE L'INVENTION

Il va à présent être décrit en relation avec les figures 1 à 8 un dispositif à coque rigide **10** conforme à un premier mode de réalisation selon l'invention, par exemple une balle de golf, une balle de hockey sur gazon, une boule de pétanque, etc...

Le dispositif **10** comprend une coque rigide **12** délimitant un volume interne **14.** Le volume interne **14** est par exemple naturellement présent dans l'objet ou est réalisé pour des objets usuellement prévus pour être pleins. La coque **12** peut donc représenter l'essentiel du volume de l'objet **10.** La paroi interne **16** de la coque **12** est par ailleurs optionnellement pourvue de picots **18,** avantageusement répartis de manière régulière sur ladite paroi **16.** Les picots sont rigides, par exemple réalisés en graphite, ou constitués de ressorts de très forte raideur, la mise en oeuvre de ressorts permettant d'augmenter la force des impacts sur l'objet interne **20.** Enfin, un objet interne **20,** par exemple sphérique, est prévu dans le volume interne **14** et peut se déplacer librement dans celui-ci. L'objet interne **20** comporte une coque **22** délimitant un volume interne **24** et constituée d'un matériau rigide et léger, comme par exemple du graphite, du plastique, un élastomère.

L'objet interne **20** comprend en outre un système de récupération d'énergie **26** qui comprend :
▪ une membrane piézoélectrique souple **28** épousant la face externe **30** de la coque **22,** avantageusement sur la totalité de celle-ci, la membrane piézoélectrique **28** libérant des charges électriques lorsqu'elle se déforme ;
▪ un circuit électrique **32,** connecté électriquement à la membrane **28** et comprenant un élément de conversion des charges électriques produites par la membrane **28** en un courant et/ou une tension constante et un ou plusieurs éléments de stockage de l'énergie électrique produite par l'élément de conversion, ainsi qu'optionnellement un circuit électronique mettant en oeuvre une ou plusieurs fonctions décrites ci-après ;
▪ un ensemble d'éléments de maintien **34** positionnant le circuit électrique **32** au centre **36** du volume interne **24** en mettant en oeuvre des forces de rappel vers ladite position, et aptes à se déformer en relation avec les déformations subies par la coque **22** afin de ne pas s'opposer à celles-ci.

Le circuit électrique **32** et l'ensemble d'éléments de maintien sont insérés le volume interne **24** de la coque interne **22.**

Ainsi, lorsque le dispositif **10** subit un impact, une accélération ou une secousse par exemple, l'objet interne **20** vient frapper la coque **12,** le cas échéant muni de picots **18,** La membrane piézoélectrique **28** plaquée contre la coque **22** subit donc une déformation et produit ainsi des charges électriques qui sont ensuite stockées et/ou traitées dans le circuit **32** comme cela est décrit ci-dessous.

De manière avantageuse, la coque **22** est constituée, ou comporte une couche, de matériau absorbant les chocs, par exemple couche de mousse, de gel, de polyester, de PCM ou de papier. Le film piézoélectrique étant placé à l'extérieur de la coque **22,** il subit pleinement les impacts de l'objet interne contre la paroi interne de la coque externe **12.** Par contre, en prévoyant une coque **22** absorbant les chocs, le circuit électrique **32** est protégé de ceux-ci.

Comme illustré aux figures 2 et 3, la membrane piézoélectrique **28,** comporte :
▪ un film piézoélectrique **38,** d'une épaisseur avantageusement comprise entre 10 micromètres et 200 micromètres, réalisé en une seule pièce ou en plusieurs pièces ;
▪ deux couches métalliques **40, 42,** d'une épaisseur de quelques nanomètres à quelques dizaines de micromètres chacune, déposées de part et d'autre du film piézoélectrique **38,** constituées par exemple d'argent, de nitrure de cuivre, d'aluminium et formant deux électrodes de collecte des charges électriques produites par le film **38** ;
▪ optionnellement un substrat flexible **44,** par exemple en plastique, tel que du polyéthylène téréphtalate (« PET ») ou du polyéthylène naphtalate (« PEN »), sur lequel est réalisé l'empilement du film piézoélectrique **28** intercalé entre les électrodes métalliques **30, 32**.

Avantageusement, le film piézoélectrique **38** est constitué de polyfluorure de vinylidène (« PVDF ») qui présente l'avantage d'être à la fois léger, souple et mécaniquement résistant, les électrodes métalliques **40, 42** pouvant être déposées directement sur les faces du film sans utiliser de substrat **44.** En variante, le film **38** est constitué de titano-zirconate de plomb (« PZT »), d'oxyde de zinc (« ZnO »), ou d'un matériau composite d'au moins deux matériaux parmi ceux-ci et le PVDF.

Le circuit électrique **22** est choisi pour être le plus léger possible compte tenu des fonctions qu'il met en oeuvre. Notamment, l'élément de stockage d'énergie électrique est avantageusement constitué d'une micro-batterie formée sur un substrat souple ou rigide. Par exemple, l'élément de stockage est une micro-batterie à substrat rigide de la gamme « EnerChip » de la société Cymbet® Corp., par exemple une micro-batterie de référence « CBC050-M8C » d'une surface de 8 x 8 mm² pour une capacité de 50 µAh, ou bien une micro-batterie à substrat souple de la société Solicore®, Inc.,par exemple un micro-batterie de référence « SF-2529-10EC » d'une surface pliable de 25,75 x 29 mm² pour une capacité de 10 mAh. En variante, l'élément de stockage d'énergie électrique comprend une ou plusieurs capacités et/ou une ou plusieurs super-capacités et/ou une ou plusieurs piles boutons rechargeables.

Le circuit **32** est également avantageusement conçu pour présenter une symétrie tridimensionnelle la plus élevée possible, l'idéal étant que le circuit **32** soit de forme sphérique et de densité uniforme. Toutefois, compte tenu des procédés usuels de fabrication des circuits électriques et électroniques, ces derniers prennent généralement une forme parallélépipédique. De manière avantageuse, le circuit **32** prend la forme d'un empilement de circuits parallélépipédiques, tel qu'illustré aux figures 4 et 5, de manière à obtenir une forme de parallélépipède rectangle, avantageusement un cube.

Le circuit **32** comporte ainsi notamment :
▪ un premier étage **46** connecté électriquement à la membrane piézoélectrique **28,** et convertissant les charges produites par celle-ci, essentiellement sous la forme d'un courant non constant, en un courant constant et/ou une tension constante, usuellement utilisée pour charger une micro-batterie, comme par exemple un circuit de type « LTC3588 » de la société Linear Technolgy Corp.,
▪ un second étage **48,** connecté électriquement au premier étage **36,** comprenant une micro-batterie se chargeant grâce au courant et/ou à la tension constante produite par le premier étage,
▪ et optionnellement un ou plusieurs troisièmes étages **50** connectés électriquement à la batterie du second étage **48** pour leur alimentation électrique, et mettant en oeuvre une ou plusieurs fonctions électroniques comme cela sera décrit plus en détail par la suite, ou comprenant une ou plusieurs éléments de stockage d'énergie électrique supplémentaires.

Les étages sont par ailleurs solidarisés au moyen d'une armature rigide **52** à laquelle sont fixés les éléments de maintien **34.**

Les éléments de maintien **34** ont quant à eux une forme longiligne, et chacun des éléments **34** est fixé à une première extrémité au circuit électrique **32,** notamment à l'armature **52** de celui-ci. De manière avantageuse, l'autre extrémité de l'élément **34** est posée contre la paroi interne de la coque interne **22,** sans être fixé de manière solidaire à cette dernière. Par exemple, les éléments **34** sont montés en compression dans la coque interne **22.**

Les éléments **34** sont fixés à l'armature du circuit **42** et à la paroi interne de la coque interne par collage, par soudure, par contact magnétique, par visserie, par un système autobloquant ou au moyen d'un système de type « *quickconnect* ». En variante, la fixation est réalisée à l'aide d'un matériau polymère, comme par exemple un polyuréthane, une colle époxy, une colle anaérobie comportant un mélange d'un diméthacrylate de glycol avec une quantité minoritaire de peroxyde et d'accélérateur de prise, un cyanoacrylate, ou un mastic MS polymère à base de silane modifié. En variante, la fixation est réalisée à l'aide de nanofibres, par exemple des nanofibres de collagène, des nanofibres de carbone et de cuivre, des nanofils de SiC comprenant des micro-pointes en carbone.

Le circuit **32** et les éléments de maintien **34,** forment ainsi avantageusement, mais optionnellement, un seul et même objet, ce qui facilite son installation dans l'objet interne **20** ainsi que son retrait.

Comme illustré à la figure 4, les éléments de maintien **34** sont avantageusement constitués de ressorts, un ressort présentant une force de rappel importante tout en étant creux, et donc léger. Par exemple, les ressorts sont constitués d'un acier inoxydable ou non, notamment un acier inoxydable AlSl302 ou AlSl316, d'alliage de nickel et de chrome, par exemple de l'inconel® 600, 625 ou 718, de cuivre ou de béryllium.

En outre, les ressorts sont choisis pour être déformable selon leur axe principal de rappel et sensiblement plus rigides perpendiculairement à cet axe, ce qui facilite la mise en contact de leur second extrémité avec la coque **22.** Dans le cadre d'un circuit électrique **32** présentant la forme d'un parallélépipède, le nombre de ressorts est avantageusement au nombre de huit, un ressort étant prévu pour chaque coin de circuit **32.** En variante, comme illustré à la figure 5, les éléments de maintien comprennent également une tige rigide **54,** positionnée entre le circuit **32** et les ressorts, afin de rigidifier le système **26** et donc rendre ce dernier plus robuste mécaniquement. Selon une variante de l'invention, les éléments de maintien **34** comprennent également un matériau piézoélectrique, ce qui permet également de récupérer de l'énergie lors de leur déformation.

Les éléments de maintien **34** présentent une force de rappel en étirement et/ou en compression choisie de manière à ce que le circuit **32** puisse se déplacer dans le volume interne **24** de l'objet **20** sans jamais impacter la paroi interne **30** sous l'effet des chocs violents que l'objet **20** est susceptible de subir lors de l'utilisation du dispositif **10.**

Avantageusement, les éléments de maintien **34** comprennent chacun plusieurs ressorts **34a, 34b,** par exemple deux, mis en série, comme illustré à la figure 6, ou en parallèle, comme illustré à la figure 7, ce qui permet de définir de manière plus aisée un comportement différent des éléments **34** en fonction de l'intensité de l'impact subi par la coque **22.** Notamment, en prévoyant plusieurs ressorts différents, il est possible de concevoir de manière simple des éléments de maintien **34** à la fois peu rigides, c'est-à-dire capables d'absorber l'énergie mécanique d'un choc afin de protéger le circuit **32,** et suffisamment rigides, c'est-à-dire évitant la collision du circuit **32** sur la coque 22 lors des impacts subis par la coque **22.**

Les figures 8, 9 et 10 illustrent des variantes de connexions électriques entre la membrane piézoélectrique **28** et le circuit électrique **32** pour transmettre à ce dernier les charges électriques produites par la membrane.

Selon une première variante illustrée à la figure 8, les deux électrodes **40, 42** de la membrane **28** sont connectés aux circuits **32,** notamment son circuit de conversion en courant/tension constant **46,** au moyen de deux fils conducteurs **62, 64** soudés auxdites électrodes et à deux plots **66, 68** du circuit **32.** Un ou deux trous sont réalisés dans la coque **22** de manière à faire passer les fils au travers de celle-ci et pouvoir connecter les fils **62, 64** aux électrodes **40, 42,** ou bien la coque **22** comporte dans son épaisseur deux plots conducteurs auxquels sont respectivement connectés les électrodes **40, 42** et les fils conducteurs **62, 64** sont connectés respectivement auxdits plots. Dans cette variante, les fils **62, 64** sont libres d'être positionnés indépendamment des éléments **34** et de l'armature **52.**

Selon une seconde variante illustrée à la figure 9, deux des éléments de maintien 34 sont conducteurs de l'électricité et sont connectés, au travers de la coque **22,** par exemple par soudure, aux électrodes **40, 42** et à des portions conductrices du circuit **32** formant les entrées électriques du circuit **32,** notamment du circuit de conversion **46.**

Selon une troisième variante, illustrée à la figure 10, les éléments **34** sont creux, par exemple constitués de ressorts, et la connexion est réalisée par deux fils conducteurs **70, 72** logés dans deux des éléments **34,** et fixés au travers de la coque **22,** par exemple par soudure, aux électrodes **40, 42** de la membrane **28** et le circuit **32,** par exemple à des plots de celui-ci ou à des portions conductrices de l'armature **52** formant des entrées électriques du circuit **32,** notamment le circuit de conversion **46.**

La première variante présente l'avantage de pouvoir choisir une armature indépendante de la connexion entre la membrane et le circuit **32.** En revanche, les fils subissent pleinement les accélérations de la balle lors des impacts de celle-ci, ce qui les fragilise.

La seconde variante propose au contraire des connexions peu sensibles auxdites accélérations, mais nécessite en contrepartie une armature plus complexe pour le circuit **32.**

La troisième variante représente un compromis entre les deux premières variantes, les fils étant protégés par les éléments **34,** et la connexion au circuit **32** pouvant être indépendante de l'armature, par exemple en prévoyant une portion de fils disposée hors des éléments **34** pour une connexion à des plots du circuit **22.**

Evidemment, ces variantes peuvent être combinées. De même, plus de deux connexions peuvent être prévues. Par exemple, dans le cas d'une membrane piézoélectrique **38** comprenant plusieurs portions électriquement isolées les unes des autres, ou membrane « pixélisée », deux connexions électriques peuvent être prévues pour chacune des portions de la membrane piézoélectrique.

Le circuit électrique **32** peut par exemple comprendre un ou plusieurs circuits électroniques alimentés en énergie électrique par la micro-batterie du circuit **32** et réalisant une fonction de traitement des impulsions électriques produites par la membrane piézoélectrique et de production de données. Ainsi, le circuit **32** peut comprendre un circuit de comptage du nombre d'impulsions produites depuis la mise en service du dispositif **10,** de détermination de l'intensité moyenne ou individuelle des impulsions, et/ou de détermination de la durée moyenne ou individuelle des impulsions. Les données ainsi produites sont par exemple stockées dans une mémoire interne du circuit **32** et/ou transmises par un circuit de transmission sans fil, par exemple par radiofréquence, du circuit **32** à l'extérieur du dispositif **10** pour leur collecte. Notamment, la connaissance du nombre d'impulsions permet de connaître, outre le nombre d'impacts subis par le dispositif **10,** l'état d'usure de ce dernier, puisque cet état d'usure dépend pour une balle, notamment directement de ce nombre. Le nombre d'impacts, leur intensité et leur durée constituent en outre des données statistiques utiles pour un joueur qui peut ainsi connaître la force de ses frappes et le type de frappes qu'il applique au dispositif, etc...En outre, en traitant les impulsions produites par chaque portion d'une membrane pixélisée, il est possible de préciser les caractéristiques des frappes, leur forme et leur empreinte sur le dispositif. De plus, l'analyse des impulsions produites par la membrane piézoélectrique permet également d'estimer la vitesse du dispositif **10.**

Lors du recyclage du dispositif **10,** les moyens de stockage d'énergie électrique du circuit **32** peuvent être déchargés pour récupérer l'énergie stockée. En outre, l'objet interne **20** peut être réutilisé dans un autre dispositif.

Une fois l'objet **20** jugé hors d'usage, il suffit pour récupérer le circuit **32** d'ouvrir la coque **22.** Lorsque les éléments de maintien **34** ne sont pas fixés à la coque **22,** il suffit alors de saisir le circuit **32** pour l'ôter de la coque **22.**

L'intégration de l'objet interne **20** dans la coque externe **12** consiste alors à prévoir la coque externe en deux demi-coques, chacune tapissée par exemple d'une membrane recouverte de picots **18,** à loger l'objet interne **20** dans l'une des deux demi-coques puis à assembler les deux demi-coques formant la coque externe **12,** par exemple par collage.

Il a été décrit un système de récupération d'énergie **26** muni d'une membrane piézoélectrique **28** souple, et donc capable d'épouser une surface non plane, et notamment sphérique. La géométrie de la coque **22** de l'objet interne **20** peut ainsi être choisie indépendamment du matériau piézoélectrique, et notamment choisi pour l'objet soit sensible aux chocs dans toutes les directions. Par contre, les matériaux piézoélectriques souples présentent usuellement des performances piézoélectriques inférieures à celles des matériaux piézoélectriques rigides.

Selon un second mode de réalisation de l'invention, le matériau piézoélectrique utilisé dans l'objet interne **20** est rigide, et constitue la totalité ou la majorité d'une coque creuse dans laquelle le circuit électrique **32** est logé.

Selon une variante illustrée aux figures 11 et 12, une coque piézoélectrique **80,** comprend une couche de matériau piézoélectrique rigide, de préférence en PZT, d'une épaisseur comprise entre quelques dizaines de nanomètres à quelques centaines de micromètres et intercalée entre deux couches métalliques formant des électrodes. La coque **80** forme un boitier en forme de parallélépipède rectangle pour le circuit électrique **32,** ce dernier étant solidarisé à la coque **80** par exemple par collage, par soudure, par contact magnétique, par visserie, par un système autobloquant ou au moyens d'un système de type « *quickconnect* ». La coque **80** constitue donc un étage supplémentaire du circuit électrique **32,** l'étage du circuit comportant les convertisseurs étant connecté à cet étage supplémentaire à l'aide de connexions appropriées, par exemple deux fils conducteurs.

Cette variante présente l'avantage d'être de construction simple. Par contre, la géométrie de la coque **80** ne permet pas d'être sensible de manière identique dans toutes les directions. En variante, la coque **80** est sphérique et remplace par exemple la coque **22** et le film piézoélectrique **28** du mode de réalisation illustré aux figures 1 à 8, ce qui permet d'obtenir une sensibilité constante quelle que soit la direction. Par contre, la fabrication d'une couche de matériau piézoélectrique rigide courbée est difficile. En effet, la fabrication d'une couche de matériau piézoélectrique rigide est usuellement réalisée au moyen d'un dépôt physique ou chimique en phase vapeur, ou sol-gel, ou au moyen d'un matériau thermodurcissable, ces techniques permettant difficilement de réaliser des objets non plans. De manière avantageuse, une coque comportant une couche de matériau piézoélectrique rigide est obtenue en réalisant tout d'abord des plaques rectangulaires planes, puis en assemblant celles-ci, par exemple par collage ou par soudure, de manière à obtenir un polyèdre.

Dans la variante illustrée aux figures 13 et 14, la coque **80** est un polyèdre à huit faces dans lequel est logé et maintenu un circuit électrique **32,** par exemple le circuit électrique maintenu par des éléments de maintien **34** tels que décrits en relation avec le premier mode de réalisation des figures 1 à 8. En variante, le circuit électrique **32** peut être fixé directement sur la coque **80** ou des éléments de maintien rigide peuvent être utilisés.

De manière avantageuse, le polyèdre est choisi pour avoir un nombre de faces important de manière à approximer une sphère, comme par exemple illustré à la figure 15, ce qui permet d'obtenir une sensibilité sensiblement indépendante de la direction.

Il a été décrit des modes de réalisation dans lesquels le matériau piézoélectrique est incorporé dans la coque d'un objet frappant la paroi interne de la coque externe du dispositif. De manière avantageuse, la paroi interne de la coque externe du dispositif est également utilisée pour convertir l'énergie mécanique en charges électriques, cette paroi étant également sujette aux chocs de la part de l'objet interne, mais également sujette à des ondes acoustiques en provenance de la paroi externe de la coque externe lorsque cette dernière subit des chocs, des frappes, des rebonds, des secousses, ou autres.

Dans un mode de réalisation illustré à la figure 16, un dispositif conforme à l'invention comporte une coque externe **12,** par exemple celle décrite en relation avec la figure 11, logeant un objet interne **20,** par exemple l'un quelconque des objets internes décrits précédemment. La paroi interne **16** de la coque externe **12** est en outre recouverte d'une membrane piézoélectrique souple **82,** par exemple telle que décrite en relation avec le premier mode de réalisation de la figure 1, de manière avantageuse y compris sur les picots **18,** et un second circuit électrique **84** est fixé à la paroi interne **16** de la coque **12,** connecté à la membrane piézoélectrique **82** et comportant des moyens souple ou non de conversion et de stockage des charges électriques produites par cette dernière, par exemple les moyens de stockage et de conversion décrits en relation avec la figure 1. En variante, la coque **12** intègre un élément piézoélectrique rigide.

Il a été décrit des modes de réalisation dans lesquels l'objet interne **20** est totalement libre de se déplacer dans le volume **14** de la coque externe **12,** l'objet interne **20** ne comportant aucun lien ni connexion avec la coque externe **12.**

De manière avantageuse, un système de rappel de l'objet interne **20** dans une position prédéterminée du volume interne **14,** par exemple le centre de celui-ci lorsque le volume est sphérique est prévu de manière supplémentaire. Par exemple, en se référant à la figure 17 qui illustre un dispositif comportant les éléments décrits en relation avec les figures 1 à 8, des ressorts **90** sont également prévus entre la coque externe **12** et la coque interne **22** de manière à appliquer une force de rappel qui maintient l'objet interne **20** au centre du volume **14** lorsque le dispositif est au repos. La raideur des ressorts **90** est choisie pour s'opposer au minimum aux déplacements de l'objet **20** tout en appliquant une force suffisante pour maintenir l'objet interne **20** au centre du volume **14** lorsque le dispositif est au repos. De cette manière, l'objet **20** est placé de sorte qu'il présente une course minimale dans le volume interne. Ainsi, quel que soit la direction de l'impact, de l'accélération ou des secousses subis par le dispositif, il existe une charge électrique minimale produite.

L'invention s'applique également aux balles, ballons, palets rigides, c'est-à-dire ne subissant sensiblement pas de déformation lors de leur utilisation, comme par exemple des balles de golf, des balles de polo, des balles de hockey sur gazon, des palets de hockey, des balles de baseball, des boules de pétanques ou autres.

L'invention s'applique également à d'autres types de sport que les sports de balles/ballons/palets/boules. Par exemple, l'invention s'applique au cyclisme, notamment en prévoyant des pédales de bicyclette comportant une cavité dans laquelle est logée un objet interne libre de s'y déplacer.

De même, l'invention s'applique également aux chaussures, par exemple de sport, comprenant par exemple des semelles munies d'une cavité et d'un objet interne conformes à l'invention.

L'invention s'applique également aux sports nautiques. Par exemple, un mat, une voile, une rame d'aviron et de kayak sont munis d'une cavité et d'un objet interne conformes à l'invention. Ainsi, à chaque fois que les objets décrits précédemment subissent un choc ou une accélération, des charges électriques sont produites, stockées et traitées, ce qui permet de fournir des statistiques par exemple.

Evidemment, l'invention s'applique à d'autres types d'activité que le sport, notamment les activités de rééducation physique qui utilisent des balles, des ballons, des boules, des palets ou autres, les statistiques produites par de tels objets conformes à l'invention permettant au personnel médical d'étudier par exemple la qualité des exercices suivis par les patients.

L'invention s'applique également aux dispositifs électroniques portables, par exemple des montres, des lecteurs MP3, des lampes à LED, ou autres, à des pièces mécaniques dans les transports (voiture, avion, vélo, revêtements de route...), des ustensiles de cuisine, des matériels en usine (alimentaire, bio), qui peuvent être rechargés par secousse en prévoyant une cavité et un objet interne conformes à l'invention.

## Revendications

1. Dispositif comportant une coque rigide externe (12) délimitant un volume interne creux (14), ***caractérisé* en ce qu'**il comprend :
▪ une coque interne (22) délimitant un volume interne creux (24), ladite coque interne (22) étant logée dans le volume interne (14) de la coque externe (12) et libre de se déplacer dans celui-ci, et comprenant au moins une couche de matériau piézoélectrique (28) apte à produire de l'énergie électrique sous l'effet d'un choc que celle-ci subit contre la coque externe (12);
▪ un circuit électrique (32) logé dans le volume interne (24) de la coque interne (22), connecté électriquement à la couche de matériau piézoélectrique (28), et comportant un élément de stockage de l'énergie électrique produite par la couche de matériau piézoélectrique (28) de la coque interne (22); et
▪ des éléments de maintien (34) du circuit électrique (32) dans la coque interne (22).

2. Dispositif selon la revendication 1, ***caractérisé* en ce que** la paroi interne (16) de la coque externe (12) est munie de picots rigides (18) régulièrement disposés sur ladite paroi.

3. Dispositif selon la revendication 1 ou 2, ***caractérisé* en ce que** la coque interne (22) est rigide.

4. Dispositif selon la revendication 3, ***caractérisé* en ce que** la coque interne rigide (22) est constituée d'un matériau piézoélectrique rigide.

5. Dispositif selon la revendication 3, ***caractérisé* en ce qu'**une membrane piézoélectrique souple (28) est plaquée contre la paroi externe (30) de la coque interne rigide (22).

6. Dispositif selon l'une quelconque des revendications 3 à 5, ***caractérisé* en ce que** la coque interne rigide (22) forme un boitier (80) pour le circuit électrique (32), et **en ce que** les éléments de maintien sont des éléments de fixation solidarisant le circuit électrique au boitier.

7. Dispositif selon l'une quelconque des revendications 3 à 5, ***caractérisé* en ce que** le circuit électrique (32) comporte une structure rigide (52), et **en ce que** les éléments de maintien (34) comportent des éléments élastiques longilignes aptes à maintenir le circuit électrique dans une position prédéterminée (26) dans le volume interne (24) de la coque interne (22), chaque élément longiligne de maintien (34) étant disposé entre la structure rigide (52) du circuit électrique (32) et une paroi interne (30) de la coque interne (22).

8. Dispositif selon la revendication 7, ***caractérisé* en ce que** les éléments de maintien (34) sont constitués de ressorts.

9. Dispositif selon l'une quelconque des revendications précédentes, ***caractérisé* en ce qu'**au moins deux des éléments de maintien (34) sont conducteurs de l'électricité et forment deux connexions électriques entre la couche piézoélectrique (28) de la coque interne (22) et le circuit électrique (32).

10. Dispositif selon l'une quelconque des revendications précédentes, ***caractérisé* en ce que** le circuit électrique (32) est constitué d'étages électriques parallélépipédiques (46, 48, 50) agencés en parallèles dans un cadre rigide (52).

11. Dispositif selon l'une quelconque des revendications précédentes, ***caractérisé* en ce qu'**il comporte une membrane piézoélectrique (82) plaquée contre la paroi interne (16) de la coque externe (12) et un circuit électrique (84) fixé à la paroi interne (16) de la coque externe (12), connecté électriquement à la membrane piézoélectrique (82) de la coque externe (12), et comportant un élément de stockage d'énergie électrique produite par la membrane piézoélectrique (82) de la coque externe (12).

12. Dispositif selon l'une quelconque des revendications précédentes, ***caractérisé* en ce que** le circuit électrique (32) comporte un circuit de production de données à partir de l'énergie électrique produite par la couche piézoélectrique de la coque interne, et un circuit de transmission sans fil desdites données à l'extérieur de la coque externe, lesdits circuits de production et de transmission étant alimentés par l'élément de stockage d'énergie électrique.

13. Dispositif selon l'une quelconque des revendications précédentes, ***caractérisé* en ce que** l'élément de stockage de l'énergie électrique comporte une micro-batterie formée sur un substrat souple ou rigide et/ou une capacité et/ou une supercapacité ou piles boutons.

14. Dispositif selon l'une quelconque des revendications précédentes, ***caractérisé* en ce que** la coque interne (22) est connectée à la coque externe (12) au moyen d'un système de rappel (90) de la coque interne (22) dans une position prédéterminée du volume interne (14) de la coque externe (12).

15. Dispositif selon l'une quelconque des revendications précédentes, ***caractérisé* en ce que** le dispositif est une balle, un ballon, un palet, une pédale de bicyclette, une semelle de chaussure, une rame, une voile, une crosse, une batte ou une raquette.

## Patentansprüche

1. Vorrichtung mit einer starren Außenschale (12), die einen hohlen Innenraum (14) definiert ***dadurch gekennzeichnet, dass*** sie umfasst:
▪ eine Innenschale (22), die einen hohlen Innenraum (24) definiert, diese Innenschale (22) ist im Innenraum (14) der Außenschale (12) untergebracht und ist dort frei verschiebbar und sie enthält mindestens eine Schicht aus piezoelektrischem Material (28), das in der Lage ist, unter der Wirkung eines Aufpralls auf die Außenschale (12) elektrische Energie zu erzeugen.
▪ einen elektrischen Schaltkreis (32), untergebracht im Innenraum (24) der Innenschale (22), elektrisch verbunden mit der piezoelektrischen Materialschicht (28) und ein Element zum Speichern der elektrischen Energie enthaltend, die durch die piezoelektrische Materialschicht (28) der Innenschale (22) erzeugt wird; und
▪ Halteelemente (34) für den elektrischen Schaltkreis (32) in der Innenschale (22).

2. Vorrichtung nach Anspruch 1, ***dadurch gekennzeichnet, dass*** die Innenwand (16) der Außenschale (12) mit starren Noppen (18) versehen ist, die regelmäßig auf dieser Wand angeordnet sind.

3. Vorrichtung nach Anspruch 1 oder 2, ***dadurch gekennzeichnet, dass*** die Innenschale (22) starr ist.

4. Vorrichtung nach Anspruch 3, ***dadurch gekennzeichnet, dass*** die starre Innenschale (22) aus einem starren piezoelektrischem Material besteht.

5. Vorrichtung nach Anspruch 3, ***dadurch gekennzeichnet, dass*** eine flexible piezoelektrische Membran (28) an der Außenwand (30) der starren Innenschale (22) anliegt.

6. Vorrichtung nach irgendeinem der Ansprüche 3 bis 5, ***dadurch gekennzeichnet, dass*** die starre Innenschale (22) ein Gehäuse (80) für den elektrischen Schaltkreis (32) bildet, und dass es sich bei den Halteelementen um Befestigungselemente handelt, die den elektrischen Schaltkreis am Gehäuse befestigen.

7. Vorrichtung nach irgendeinem der Ansprüche 3 bis 5, ***dadurch gekennzeichnet, dass*** der elektrische Schaltkreis (32) eine starre Konstruktion (52) umfasst, und dass die Halteelemente (34) elastische, längliche Elemente enthalten, die in der Lage sind, den elektrischen Schaltkreis in einer vorher festgelegten Position (26) im Innenraum (24) der Innenschale (22) zu halten, wobei jedes längliche Halteelement (34) zwischen der starren Konstruktion (52) des elektrischen Schaltkreises (32) und einer Innenwand (30) der Innenschale (22) angeordnet sind.

8. Vorrichtung nach Anspruch 7, ***dadurch gekennzeichnet, dass*** die Halteelemente (34) aus Federn bestehen.

9. Vorrichtung nach irgendeinem der vorhergehenden Ansprüche, ***dadurch gekennzeichnet, dass*** mindestens zwei der Halteelemente (34) elektrische Leiter sind und zwei elektrische Verbindungen zwischen der piezoelektrischen Schicht (28) der Innenschale (22) und dem elektrischen Schaltkreis (32) bilden.

10. Vorrichtung nach irgendeinem der vorhergehenden Ansprüche, ***dadurch gekennzeichnet, dass*** der elektrische Schaltkreis (32) aus quaderförmigen elektrischen Stufen (46, 48, 50) besteht, die parallel in einem starren Rahmen (52) angeordnet sind.

11. Vorrichtung nach irgendeinem der vorhergehenden Ansprüche, ***dadurch gekennzeichnet, dass*** sie eine piezoelektrische Membran (82) enthält, die an der Innenwand (16) der Außenschale (12) anliegt sowie einen elektrischen Schaltkreis (84), befestigt an der Innenwand (16) der Außenschale (12) und elektrisch verbunden mit der piezoelektrischen Membran (82) der Außenschale (12), und dass sie ein Element zum Speichern der elektrischen Energie enthält, die durch die piezoelektrische Membran (82) der Außenschale (12) erzeugt wird.

12. Vorrichtung nach irgendeinem der vorhergehenden Ansprüche, ***dadurch gekennzeichnet, dass*** der elektrische Schaltkreis (32) einen Kreislauf zur Datenerzeugung aus der elektrischen Energie, erzeugt durch die piezoelektrische Membran der Innenschale sowie einen drahtlosen Übertragungskreislauf dieser Daten nach außerhalb der Außenschale enthält, wobei diese Kreisläufe zur Erzeugung und Übertragung vom Element zur Speicherung der elektrischen Energie versorgt wird.

13. Vorrichtung nach irgendeinem der vorhergehenden Ansprüche, ***dadurch gekennzeichnet, dass*** das Element zur Speicherung der elektrischen Energie eine Mikrobatterie enthält, die auf einem flexiblen oder starren Substrat gebildet wird und/oder einen Kondensator und/oder Superkondensator oder Knopfbatterien enthält.

14. Vorrichtung nach irgendeinem der vorhergehenden Ansprüche, ***dadurch gekennzeichnet, dass*** die Innenschale (22) mit der Außenschale (12) über ein Rückstellsystem (90) der Innenschale (22) in eine vorher festgelegte Position des Innenraums (14) der Außenschale (12) verbunden ist.

15. Vorrichtung nach irgendeinem der vorhergehenden Ansprüche, ***dadurch gekennzeichnet, dass*** diese Vorrichtung eine Kugel, ein Ballon, ein Puck, ein Fahrradpedal, eine Schuhsohle, ein Ruder, ein Segel, ein Stab, ein Schläger oder ein Racket ist.

## Claims

1. A device comprising a rigid outer shell (12) defining a hollow inner space (14), ***characterized* in that** it comprises:
▪ an inner shell (22) defining a hollow inner space (24), said inner shell (22) being housed in the inner space (14) of the outer shell (12) and capable of freely moving therein, and comprising at least one layer of piezoelectric material (28) capable of generating electric energy under the effect of an impact to which it is submitted against the outer shell (12);
▪ an electric circuit (32) housed in the inner space (24) of the inner shell (22), electrically connected to the piezoelectric material layer (28), and comprising an element for storing the electric energy generated by the piezoelectric material layer (28) of the inner shell (22); and
▪ elements (34) for holding the electric circuit (32) in the inner shell (22).

2. The device of claim 1, ***characterized* in that** the inner wall (16) of the outer shell (12) is provided with rigid spikes (18) regularly arranged on said wall.

3. The device of claim 1 or 2, ***characterized* in that** the inner shell (22) is rigid.

4. The device of claim 3, ***characterized* in that** the rigid inner shell (22) is made of a rigid piezoelectric material.

5. The device of claim 3, ***characterized* in that** a flexible piezoelectric membrane (28) is applied against the outer wall (30) of the rigid inner shell (22).

6. The device of any of claims 3 to 5, ***characterized* in that** the rigid inner shell (22) forms a package (80) for the electric circuit (32), and **in that** the holding elements are fastening elements securing the electric circuit to the package.

7. The device of any of claims 3 to 5, ***characterized* in that** the electric circuit (32) comprises a rigid structure (52), and **in that** the holding elements (34) comprise longilineal resilient elements capable of holding the electric circuit in a predetermined position (26) in the inner space (24) of the inner shell (22), each longilineal holding element (34) being arranged between the rigid structure (52) of the electric circuit (32) and an inner wall (30) of the inner shell (22).

8. The device of claim 7, ***characterized* in that** the holding elements (34) are formed of springs.

9. The device of any of the foregoing claims, ***characterized* in that** at least two of the holding elements (34) are electrically conductive and form two electric connections between the piezoelectric layer (28) of the inner shell (22) and the electric circuit (32).

10. The device of any of the foregoing claims, ***characterized* in that** the electric circuit (32) is formed of parallelepipedal electric stages (46, 48, 50) arranged in parallel in a rigid frame (52).

11. The device of any of the foregoing claims, ***characterized* in that** it comprises a piezoelectric membrane (82) applied against the inner wall (16) of the outer shell (12) and an electric circuit (84) fastened to the inner wall (16) of the outer shell (12), electrically connected to the piezoelectric membrane (82) of the outer shell (12), and comprising an element for storing electric energy generated by the piezoelectric membrane (82) of the outer shell (12).

12. The device of any of the foregoing claims, ***characterized* in that** the electric circuit (32) comprises a circuit for generating data from the electric energy generated by the piezoelectric layer of the inner shell, and a circuit of wireless transmission of said data outside of the outer shell, said generation and transmission circuits being powered by the electric energy storage element.

13. The device of any of the foregoing claims, ***characterized* in that** the electric power storage element comprises a microbattery formed on a flexible or rigid substrate and/or a capacitor and/or a supercapacitor or button cells.

14. The device of any of the foregoing claims, ***characterized* in that** the inner shell (22) is connected to the outer shell (12) by means of a system (90) for pulling back the inner shell (22) to a predetermined position of the inner space (14) of the outer shell (12).

15. The device of any of the foregoing claims, ***characterized* in that** the device is a ball, a puck, bicycle pedal, a shoe sole, an oar, a sail, a stick, a bat, or a racket.
